# FASCICULE DE BREVET EUROPEEN

(11) **EP 2 184 975 B1**
(45) Date de publication et mention de la délivrance du brevet: **28.10.2015**
(21) Numéro de dépôt: 08843538.3
(22) Date de dépôt: 12.08.2008
(51) Int. Cl.: A01K 61/00, A01K 67/033

(54) **Procédé d'obtention d'huîtres résistantes à des agents pathogènes, et huître obtenue par ledit procédé**
Verfahren zur Gewinnung von gegen Krankheitserregende Wirkstoffe resistenten Austern, und durch dieses Verfahren erhaltene Auster
Method for obtaining oysters resistant to pathogenic agents, and oyster obtained by said process

(30) Priorité: 14.08.2007 FR 0705850
(43) Date de publication de la demande: 19.05.2010
(73) Titulaire: Lebrun, Guy, 49320 Brissac Quincé (FR)
(72) Inventeur: Lebrun, Guy, 49320 Brissac Quincé (FR)
(74) Mandataire: Novagraaf Technologies
(86) Numéro de dépôt international: PCT/FR2008/001187
(87) Numéro de publication internationale: WO 2009/056705

(56) Documents cités:
- GB-A- 2 282 039
- DATABASE BIOSIS [Online] BIOSCIENCES INFORMATION SERVICE, PHILADELPHIA, PA, US; octobre 2000 (2000-10), NACIRI-GRAVEN YAMAMA ET AL: "Influence of parentage upon growth in Ostrea edulis: Evidence for inbreeding depression" XP002473367 Database accession no. PREV200100326560 & GENETICAL RESEARCH, vol. 76, no. 2, octobre 2000 (2000-10), pages 159-168, ISSN: 0016-6723
- SAMAIN ET AL: "Genetically based resistance to summer mortality in the Pacific oyster (Crassostrea gigas) and its relationship with physiological, immunological characteristics and infection processes" AQUACULTURE, ELSEVIER, vol. 268, no. 1-4, 5 juillet 2007 (2007-07-05), pages 227-243, XP022143488 ISSN: 0044-8486
- Standish K Allen ET AL: "Genetic Improvement of the Eastern Oyster for Growth and Disease Resistance in the Northeast" In: "Genetic Improvement of the Eastern Oyster for Growth and Disease Resistance in the Northeast", 1 January 1993 (1993-01-01), Northeastern Regional Aquaculture Center, Darmouth, Massachusetts, XP055106524, vol. 210

## Description

### Domaine technique

La présente invention se rapporte à un procédé d'obtention d'huîtres résistantes à des agents pathogènes et aux huîtres ainsi obtenues.

La présente invention trouve notamment une application dans la production commerciale d'huîtres en élevage résistante à des agents pathogènes tels que *Bonamia Ostreae* et *Martelia Refringens.*

Dans la description ci-dessous, les références entre crochets (**[ ]**) renvoient à la liste des références présentées après les exemples.

### État de la technique

L'huître plate *Ostrea edulis* est une des espèces d'huîtres les plus cultivée en Europe mais également aux Etats-Unis ainsi qu'au Canada.

Dans les années 70-80, l'apparition des parasites *Martelia refringens* et *Bonamia ostreae* a produit une forte diminution de la production des huîtres obligeant les ostréiculteurs à chercher des moyens afin de combattre ces parasites.

Plusieurs mesures ont été prises pour limiter l'infection des huîtres. Une des mesures fut de limiter le mouvement des huîtres d'une écloserie à une autre et de limiter le mouvement des naissains d'une région à une autre.

Une autre mesure fut d'essayer de sélectionner des huîtres résistantes au parasite *Bonamia Ostreae,* Naciri-Graven et al. (1998) « Selecting the flat oyster Ostrea edulis (L.) for survival when infected with the parasite Bonamia ostreae. » J.Exp. Mar Biol. Ecol., 224 :91-107 [1]. Ces sélections ont permis de mettre en avant des huîtres tolérantes auxdits parasites mais non résistantes et ont provoqué un affaiblissement de la diversité génétique des huîtres cultivées ainsi qu'une augmentation néfaste de la consanguinité des huîtres, N. Taris et al. « Conséquences génétiques de la production de larves d'huîtres en écloserie : étude des processus de dérive et de sélection. » , Les actes du BRG, 6 (2006) 521-541. [2].

D'autres études ont été menées afin d'identifier des zones de culture d'huîtres non infectées en utilisant des naissains sensibles provenant d'Atlantique. Une de ces études fut menée en Corse dans les années 80 dans l'étang de Diana aboutissant au classement de cet étang comme zone infectée sur proposition d'IFREMER, au titre de la directive du Conseil Européen 91/67/CEE relative aux conditions de police sanitaire régissant la mise sur le marché d'animaux et de produits d'aquaculture. La zone I qui englobe toute la Corse est classée comme non indemne pour la bonamiose et la marteliose.

Ces différentes études ont permis d'établir une cartographie des zones infectées par les parasites et même de sélectionner par zone des huîtres plus tolérantes aux parasites mais en affaiblissant leur diversité génétique.

Aucune de ces études n'a permis de produire des huîtres résistantes auxdits parasites.

Le document "Genetic Improvement of the Eastern Oyster for Growth and Disease Résistance in the Northeast", Standish K Allen et al. (1993), Northeastern Regional Aquaculture Center, Darmouth, Massachusetts, NRAC Fact Sheet No. 210, évoque un procédé d'obtention d'une huître résistante à des agents pathogènes par hybridation interspécifque d'huîtres du genre *Crassostrea*.

Il existe donc un réel besoin d'un procédé permettant d'obtenir des huîtres résistantes aux agents pathogènes palliant les défauts, inconvénients et obstacles de l'art antérieur et permettant de maîtriser une production industrielle à des coûts réduits. Ce procédé devant être simple à mettre en oeuvre.

### Description de l'invention

La présente invention a précisément pour but de répondre aux besoins et inconvénients précités en fournissant un procédé d'obtention d'huîtres résistantes aux agents pathogènes.

Le procédé de l'invention se caractérise en ce qu'il comprend l'hybridation d'une huître *Ostrea edulis* de Méditerranée résistante auxdits agents pathogènes et d'une huître non résistante, suivant la revendication 1.

L'invention se rapporte également aux huîtres résistantes à des agents pathogènes susceptibles d'être obtenues par le procédé de l'invention, suivant la revendication 19.

Selon l'invention, par huîtres résistantes on entend toute huître *Ostrea edulis* de Méditerranée résistante aux agents pathogènes. L'huître résistante *Ostrea edulis* de Méditerranée peut être toutes huîtres sauvages résistantes originaires de Méditerranée. Par exemple, toutes huîtres *Ostrea edulis* de Méditerranée n'ayant pas subies de pression sélective d'origine anthropique ni de contamination génétique, par exemple par l'élevage et/ou par la transplantation de souches cultivées provenant de l'Atlantique. L'origine méditerranéenne des huîtres peut être vérifiée par des analyses moléculaires des souches par des marqueurs allozymes, des marqueurs microsatellites, Launey S. et al. « Géographie structure in the European flat oyster (Ostrea edulis L.) as revealed by Microsatellite polymorphism. » J Hered. 2002 Sep-Oct;93(5):331-51. (2002) [3], ou des séquences ARN. Par exemple un marqueur moléculaire qui peut être utilisé afin de mettre en évidence l'origine méditerranéenne de la couche correspond à la séquence de 313 paires de bases du gène 12s-rRNA mitochondrial des souches tel que présenté dans l'article scientifique Diaz-Almela et al. « Reduced female gene flow in the European flat oyster Ostrea edulis ». J Hered. 2004 Nov-Dec;95(6):510-6. (2004) [4].

L'huître résistante peut être choisie de manière à présenter, de préférence, des qualités zootechniques de croissance et des indices de condition, c'est-à-dire une proportion de chair comestible par rapport au poids total, les plus adaptés au procédé d'hybridation. Par exemple, ladite proportion peut être comprise entre 0.10 et 0.20, de préférence supérieurs à 0.15. La mesure peut-être réalisée par exemple sur les huîtres les plus grosses au sein d'une classe d'âge et présentant une conformation de coquille, c'est-à-dire une convexité de valve inférieure, adaptée. L'huître résistante peut être choisie, par exemple parmi des huîtres jeunes de première maturité, par exemple d'un poids inférieur à 80g pour l'huître de Corse, les huîtres plus grosses ayant tendance à produire une proportion supérieure de coquille. Parmi ceux-ci, on peut retenir de préférence les individus présentant une convexité, définie comme étant le rapport de l'épaisseur sur le diamètre moyen [(longueur+largeur)/2], supérieure à 0,33.

Les huîtres résistantes peuvent êtres choisies en fonction de leurs caractéristiques phénotypiques telle que la taille, le poids etc. Par exemple, l'huître *Ostrea edulis* résistante de Méditerranée peut être choisie, de préférence, avec un diamètre supérieur à 60mm.

Les huîtres résistantes peuvent également êtres choisies en fonction de leur localisation au niveau du milieu naturel de croissance. Par exemple, entre les formes libres, disséminées à la surface du sédiment et les huîtres fixées sur des parties rocheuses dans leur milieu naturel, les formes libres sont préférées car elles présentent des caractéristiques plus adaptées pour l'élevage.

Les zones de prélèvement des huîtres au niveau des sites naturels sont de préférence celles comportant une population la plus importante, c'est-à-dire avec une productivité la plus grande. Pour les formes libres occupant le sédiment meuble, les huîtres peuvent être choisies, par exemple sur les peuplements ayant une densité supérieure à 1 individu par m², et, de préférence supérieure à 2 individus par m².

L'huître *Ostrea edulis* résistante peut être choisie, par exemple, parmi les huîtres *Ostrea edulis* résistantes de Méditerranée, des huîtres provenant des lagunes de Corse, du Maroc à Nador, de Tunisie à Gabes, de Libye, de Grèce, de Murcie dans la Mar Menor en Espagne, de la région égéenne et du Bosphore en Turquie, de Minorque aux Baléares, d'Ukraine à Sévastopol, de la Mer Rouge et/ou de Mer Baltique.

De préférence, pour la réalisation du procédé de l'invention, l'huître *Ostrea edulis* résistante est choisie dans le groupe comprenant l'huître de Corse *Ostrea edulis* sauvage de l'étangs de Diane, de l'étangs d'Urbino, du golfe de Porto Vecchio, du golfe de Santa Manza et des lagunes de Sardaigne. De préférence pour la réalisation du procédé de l'invention, l'huître *Ostrea edulis* résistante choisie est l'huître de Corse *Ostrea edulis* sauvage de l'étangs de Diane.

Selon l'invention, les huîtres résistantes de Méditerranée peuvent être des huîtres provenant de gisements anciens de Méditerranée. Les huîtres sont de préférence des huîtres plates sauvages qui n'ont pas subi d'apports génétiques extérieurs issus de transplantations ou d'élevage et/ou des huîtres provenant de lagunes méditerranéennes géographiquement limitées. Les populations sauvages installées de longue date dans les lagunes de Méditerranées présentent les caractères génotypiques, phénotypiques et zootechniques propres à l'apport des qualités adaptatives particulières attachées à leur biotope, en particulier les niveaux et amplitudes des températures, les niveaux et variations de salinité, les oxygénations du milieu, les qualités trophiques du milieu, et les marées et la résistance à l'exondation possible des gisements naturels. Par exemple, les gisements naturels d'huîtres de la lagune de Nador au Maroc peuvent être exondés pendant plusieurs heures aux marées de vives eaux et soumis à l'ensoleillement subtropical de la région sans subir de mortalité. L'huître résistante peut être une huître de Nador pour la réalisation du procédé de l'invention par exemple pour obtenir un hybride adapté à l'élevage sur estran en Atlantique.

Toutefois, les souches méditerranéennes résistantes provenant de biotopes ouverts et appauvris au niveau nutritionnel héritent de caractères adaptatifs communs généralement peu propices à la qualité de l'hybridation. C'est le cas des souches résistantes provenant des Îles grecques des Cyclades, de Croatie, de Calabre, de Sicile, et de Camargue (Port-Saint-Louis-du-Rhône).

L'origine méditerranéenne des huîtres peut être vérifiée par exemple par analyses moléculaires des souches par des marqueurs allozymes, des marqueurs microsatellites [3] ou des séquences ARN [4] telles que décrites précédemment.

Selon l'invention, avantageusement l'huître résistante choisie présente un niveau de réserves glucidiques approprié à l'hybridation. Ces réserves, constituées de glycogène vont progressivement donner l'aspect d'une huître « grasse » propre à développer sa gonade et à produire ses gamètes. Ces réserves peuvent être mobilisées au printemps pour la fabrication des gamètes et pendant toute la gamétogénèse; ainsi, la nutrition des géniteurs conditionne la fécondité et le recrutement Les réserves glucidiques permettent de favoriser l'expression de la pleine fécondité des huîtres et donc la réalisation du procédé de l'invention.

Selon l'invention, l'huître *Ostrea edulis* résistante de Méditerranée est de préférence choisie à un âge de reproduction, de préférence à l'âge de première reproduction. Le choix de l'huître à l'âge de première reproduction permet en effet de garantir un taux de survie des gamètes maximum pour la réalisation de l'hybridation.

Selon l'invention, par huître non résistantes on entend toute huître sensible, vulnérable aux agents pathogènes.

La non résistance de l'huître aux agents pathogènes peut se manifester par toute altération des fonctions biologiques de l'huître pouvant entraîner par exemple une croissance modifiée, diminuée, ou arrêtée de l'huître. La non résistance aux agents pathogènes peut également conduire à la mort de l'huître. Dans le cas de la bonamiose, la première indication d'infection survient souvent à la suite de ralentissement de croissance, de lésions sur les branchies, d'ouverture des valves, accompagnée de fortes mortalités apparaissant généralement à partir de la première maturation sexuelle. Pour la marteliose, dite « Maladie des Abers », les signes cliniques suivants apparaissent dans les élevages en abers ou sur estran : émaciation et épuisement des réserves de glycogène, décoloration complète de la glande digestive, arrêt du nourrissage et affaiblissement de l'huître, signes suivis de fortes mortalités.

En d'autres termes, l'huître non résistante peut être n'importe quelle huître sensible aux pathologies des huîtres cultivées. Il peut s'agir, par exemple d'une huître bivalve de la famille des Ostréidés.

Par exemple, l'huître non résistante peut-être une huître sensible au virus de l'herpes 1 des huîtres (« l'Ostreid Herpesvirus-1 (OsHV-1)»).

Par exemple, l'huître non résistante peut être une huître dont les défenses immunitaires sont affaiblies en raison de variation des facteurs environnementaux : éléments toxiques, contaminants ou facteurs physicochimiques du milieu (Gagnaire B., 2005) [10]

Par exemple, l'huître non résistante peut être aussi une huître rendue sensible aux agents infectieux par dérive génétique par suite de pratiques culturales ou de pression de sélection en écloserie (Taris N., 2007) [15]

Selon l'invention, les huîtres non résistantes pouvant être hybridées selon le procédé de la présente invention peuvent être choisies parmi les huîtres sauvages, les huîtres cultivées, issues de captage naturel ou d'écloserie, les souches d'huîtres natives, parmi les huîtres d'origines extérieures à la Méditerranée. Selon l'invention l'huître non résistante est une huître de l'Atlantique, du Pacifique et/ou de l'océan Indien.

Selon l'invention, l'huître non résistante peut être d'un genre ou d'une espèce identique ou différent de l'huître *Ostrea edulis* résistante. Il peut s'agir, par exemple d'une une huître plate ou creuse. Les espèces d'huîtres non résistantes qui peuvent être utilisées dans l'invention appartiennent aux genres et/ou aux espèces *Ostrea edulis, Ostrea angasi, Ostrea conchaphila, Ostrea. lurida, Ostrea denselamellosa, Ostrea puelchana, Ostrea folium, Ostrea permollis, Ostrea stentina, Tiostrea chilensis, Crassostrea virginica, Crassostrea gasar* et *Crassostrea Rhizophorae,* etc.

L'huître non résistante peut être choisie, par exemple, dans le groupe comprenant *Crassostrea gasar* huître de palétuvier, des estuaires et mangroves d'Afrique de l'Ouest, *Crassostrea Rhizophorae* huître de palétuvier, indo pacifique etc.

L'huître *Ostrea edulis* non résistante peut être choisie, par exemple, dans le groupe comprenant des huîtres plates « gravettes » provenant du Bassin d'Arcachon, de Charente et/ou de Vendée, l'huître plate de Bretagne Sud la « Belon » provenant d'Abers et du golfe du Morbihan, des « Cancales » provenant de Bretagne nord et de Normandie, des huîtres provenant de Galice en Espagne, des huîtres provenant du Portugal, des huîtres de Grande Bretagne de Cornouailles, des huîtres du sud et du nord de l'Irlande, des huîtres des Pays-Bas, des huîtres du Danemark, des huîtres des USA et du Canada de l'Atlantique Ouest, de Floride au Québec, des huîtres du Pacifique Est provenant des cotes des USA et du Canada, des huîtres des côtes allant de la Californie à l'Alaska.

L'huître non résistante peut être infectée ou non infectée, tolérante ou non tolérante aux agents pathogènes pour la mise en oeuvre du procédé de l'invention. Ladite huître est dite tolérante lorsqu'elle est susceptible de se reproduire une première fois avant d'avoir exprimé les pathologies qu'elle porte.

Selon l'invention, pour la mise en oeuvre du procédé de l'invention, l'huître *Ostrea edulis* résistante de Méditerranée et l'huître non résistante sont de préférence choisies à l'issue de la période de repos sexuel. Cette période se situe normalement en hiver. Lesdites huîtres résistantes et non résistantes sont de préférence alors conditionnées pour l'hybridation, par exemple en bacs d'écloserie, par exemple en photopériode croissante.

Selon l'invention, les huîtres peuvent être marquées individuellement en fonction du caractère résistant ou non résistant aux agents pathogènes. Le marquage peut être réalisé par l'un quelconques des moyens connus de l'homme du métier, par exemple par scarification ou par collage d'étiquette à la colle, par exemple par colle époxy. Ce marquage individuel des huîtres permet de faciliter la constitution de lots homogènes de naissains hybrides.

Selon l'invention, les huîtres utilisées pour l'hybridation sont de sexe opposé. L'huître plate du genre *Ostrea* est un bivalve asynchrone à sexualité consécutive rythmique. Généralement protandrique, elle peut changer de sexe plusieurs fois dans la même saison, ce qui rend difficile le sexage car les lignées mâles et femelles coexistent simultanément. Ce mode de reproduction spécifique attaché au genre *Ostrea* interdit tout contrôle des identités génétiques en cas de reproduction en masse. Il est donc préférable d'isoler deux à deux les huîtres, dans le cas ou l'hybridation fait intervenir des huîtres résistantes et non résistantes du genre *Ostrea.* En d'autres termes, il est donc préférable d'isoler deux à deux les huîtres. En théorie, chaque paire d'huîtres isolées ne devrait avoir qu'une chance sur deux de former le couple mâle-femelle. Avantageusement dans la présente invention, la fécondation survient au niveau de 70 à 80% des couples. En effet, la maturation de l'huître la plus précoce peut provoquer une orientation inverse du sexe de la seconde huître. Le nombre d'hybridations obtenu par la présente invention est donc supérieur au nombre théorique supposé.

On peut noter que les huîtres sont largement eurythermes et euryhalins. Ces tolérances facilitent la synchronisation des maturations et de l'émission de gamètes, et l'hybridation de deux huîtres issues de milieux géographiques singuliers.

Selon l'invention, par « agent pathogènes » ont entend un pathogène unique ou plusieurs pathogènes ou des pathogènes responsables unique ou en combinaison avec d'autres agents, de pathologies bactériennes, virales, parasitaires ou des pathologies associées à celles-ci.

Selon l'invention, les agents pathogènes peuvent être choisis dans le groupe comprenant les haplosporidies, les protozoaires, les martelias, les parasites et les agents contaminants externes faisant appel aux mécanismes généraux d'immunité cellulaire et/ou humorale des huîtres, par exemple les agents pathogènes *Bonamia ostreae* et *Martelia refringens.*

Selon l'invention, lesdits agents pathogènes peuvent êtres responsables de pathologies, par exemple, la bonamiose, la marteliose, l'haplosporidose, la microcytose, perkinsose et/ou la irivovirose, de préférence les agents pathogènes sont responsables de la bonamiose et/ou de la martéliose

Selon l'invention, lesdits agents pathogènes peuvent être responsables d'épizooties, ou de syndromes de mortalités massives ou récurrentes par exemple de mortalités estivales associées par exemple à l'augmentation de la température du milieu de culture et/ou au virus de l'herpes 1 de l'huître (« l'Ostreid Herpesvirus-1 (OsHV-1)») et à des agents opportunistes par exemple *Nocardia crassostreae,* Ces agents pathogènes et/ou opportunistes décrits chez les mollusques d'intérêt économique sont regroupés dans le « Synopsis of Infectious Diseases and Parasites of Commercially Exploited Shellfish » (Bower et al., 1994) [13], (Bower & McGladdery, 2003) [14]

Des moyens de mise en oeuvre du procédé de la présente invention sont décrits ci-après.

Selon l'invention, l'hybridation peut être réalisée dans toute cuve connue de l'homme du métier permettant la réalisation de l'hybridation des huîtres. Par exemple la cuve peut-être un bac d'éclosion, un aquarium, toute cuve, bac, bassin en verre, bois, métal, matière synthétique ou maçonnerie, ou tous récipients adaptés. Le volume de la cuve utilisée pour la réalisation du procédé de l'invention peut être par exemple d'un volume de 1 à 100 litres, de préférence de 5 à 20 litres.

Selon l'invention, la cuve peut comporter des moyens d'oxygénation de l'eau. Par exemple, l'eau peut être oxygénée à l'aide de tous les moyens connus de l'homme du métier, par exemple par une pompe permettant l'expulsion de l'air dans l'eau de la cuve. L'oxygénation peut être également effectué au niveau du circuit d'eau. Le débit d'oxygénation de l'eau peut être adapté au cours de la réalisation de l'hybridation, de préférence de façon à saturer le milieu aquatique en oxygène dissous, quels que soient les niveaux de température et de salinité requis selon l'invention. Le niveau d'oxygène peut être de 5 mg/l à 11 mg/l, de préférence supérieur à 6,5 mg/l. Selon l'invention, la cuve comprenant les huîtres à hybrider peut être, par exemple préalablement remplie avec de l'eau, alimentée en continu ou en discontinu par un circuit d'eau. Le débit de circulation d'eau peut être compris entre 0 l/h et 100 l/h, de préférence entre 5 l/h et 20 l/h. Le débit peut être et ajusté aux besoins de respiration et de nourrissage des huîtres et des larves.

Selon l'invention, ledit circuit d'eau peut être isolé d'une cuve à une autre. L'isolement du circuit d'eau permet notamment d'éviter des influences extérieures sur les huîtres à hybrider, et de garantir ainsi la pureté des larves hybrides. Cet isolement permet par exemple de prévenir la production des hybrides de contaminations génétiques indésirables.

Selon l'invention, l'eau utilisée dans la présente invention peut être toute eau adaptée à la réalisation de l'hybridation. L'eau adaptée à la réalisation de l'invention peut être, par exemple, une eau présentant des caractères physicochimiques, par exemple une salinité, un pH, une température etc., identiques, intermédiaires ou différents de ceux du milieu d'origine des huîtres non résistantes et/ou de ceux du milieu des huîtres résistantes.

Selon l'invention, l'eau peut comporter une composition différente en fonction des étapes du procédé de l'invention.

Selon l'invention, l'eau utilisée peut avoir une salinité comprise entre 3 ‰ et 50 ‰, de préférence entre 12 ‰ et 38 ‰. Elle peut avoir un pH compris entre 6,5 et 9, de préférence entre 7 et 8,5. Elle peut être à une température comprise entre - 8°C et + 55°C, de préférence entre 12°C et 40°C.

Selon l'invention, l'apport nutritionnel pour la réalisation de l'invention peut être constitué par toute source nutritionnelle connue de l'homme du métier pour la culture d'huître et/ou pour l'hybridation d'huître. Ledit apport nutritionnel peut être constitué, par exemple d'algues unicellulaires, de phytoplanctons, de bactéries benthiques d'apports de microparticules alimentaires, d'aliments composés, d'apports de matières organiques dissoutes tel que des sucres, des protéines, des vitamines etc. ou d'un mélange de ceux-ci.

Le phytoplancton, les bactéries benthiques ainsi que les algues unicellulaires peuvent provenir d'écloserie et/ou de milieux naturels de croissance des huîtres. Les microparticules peuvent être d'origine naturelle ou artificielle, par exemple issues de déchets organiques et/ou de rejets aquacoles.

Les algues unicellulaires et/ou le phytoplanctoon peuvent être par exemple concentrés par centrifugation sous forme de pâte réfrigérée, les microparticules alimentaires peuvent être par exemple en suspension et/ou en émulsions, les aliments composés peuvent être par exemple secs ou hydratés, associant des matières glucidiques et/ou lipidiques et/ou protéiques, éventuellement extrudés, calibrés ou en microcapsules.

Les algues unicellulaires cultivées en écloseries et/ou en milieu naturel peuvent être choisies dans le groupe comprenant *Isochrysis galbana, Skeletonema costatum, Pavlova lutheri, Chaetoceros calcitrans, Tetraselmis sp.* etc. Par exemple, le phytoplancton peut être choisi dans le groupe comprenant : *Isochrysis galbana, Pavlova lutheri, Chaetoceros forma pumilum* etc.. La quantité et la qualité du phytoplancton et des algues unicellulaires utilisées sont de préférence adaptées à la réalisation du procédé de l'invention. La nutrition est un élément important pour la fécondité des géniteurs et le succès du recrutement en larves et huîtres hybrides.

Dans la présente, on entend par « hybridation » toutes les méthodes connues de l'homme du métier permettant la réalisation d'une hybridation entre au moins une huître résistante et une huître non résistante telles que décrites précédemment. L'hybridation peut également être réalisée directement avec les gamètes des huîtres résistantes et/ou non résistantes décrites précédemment par les techniques de cryoconservation du sperme selon les travaux de Lannan J.E, 1971, Expérimental self-fertilization of the Pacific oyster, Crassostrea gigas, utilising cryopreserved sperm, Genetics, 68 : 599-601 [5], Bougrier et Rabedomanana ,1986. Cryopreservation of spermatozoa of the Japanese oyster, Crassostreea gigas. Aquaculture, 58 : 277-280 [6], ou au moyen des dilueurs de conservation et de lavage multispécifiques des gamètes Haffray et al. « Domestication et amélioration génétique des cheptels piscicoles dans le cadre du SYSAAF », INRA Prod. Anim., 2004, 17 (3), 243-252 [7]. Les connaissances zootechniques peuvent permettre de maîtriser la reproduction artificielle d'individus identifiés, par le contrôle du sexe, par la maturation individuelle des géniteurs, par le synchronisme des pontes et par les échanges chimiques entre géniteurs. Ces connaissances ont fait l'objet d'une publication scientifique de synthèse : Gerard A., Naciri-Graven Y., Boudry P., Launey S., Heutebise S., Ledu C., Phelipot P., (1997). Contrôle de la gamétogénèse des huîtres creuses et plates. In : La reproduction naturelle et contrôlée des Bivalves cultivés en France, Devauchelle N., Barret J., Salaun G., (1997), DRV/RA/RST 97-11, Ifremer Brest, 217 pp. [8].

Selon un mode particulier de réalisation de l'invention, l'hybridation peut comprendre les étapes suivantes :
a) fournir une huître *Ostrea edulis* de Méditerranée résistante auxdits agents pathogènes,
b) fournir une huître non résistante auxdits agents pathogènes du sexe opposé à ladite huître *Ostrea edulis* résistante,
c) mettre ensemble lesdites huîtres dans une cuve comprenant de l'eau ayant une salinité permettant l'hybridation,
d) maturation desdites huîtres dans ladite cuve, de préférence en photopériode croissante, avec un apport nutritionnel suffisant pour ladite maturation,
e) induction de l'émission de gamètes mâles au moyen d'un agent d'induction appliqué aux huîtres,
f) fécondation des gamètes femelles par les gamètes mâles émises à l'étape e),
g) incubation des larves obtenue à l'étape f) pendant 8 à 10 jours,
h) nourrissage des larves issues de l'étape g) par du phytoplancton de façon à obtenir des larves oeillées,
i) récolte des larves oeillées obtenues, et
j) fixation desdites larves sur un support de développement afin d'obtenir au moins une huître résistante auxdits agents pathogènes.

Selon le procédé de l'invention, l'huître *Ostrea edulis* de Méditerranée résistante aux agents pathogènes fournie à l'étape a) est une huître résistante telle que décrite précédemment.

Selon le procédé de l'invention, l'huître non résistante aux agents pathogènes fournie à l'étape b) est une huître non résistante telle que décrite précédemment.

Selon le procédé de l'invention l'étape c) consiste à mettre ensemble au moins une huître fournie à l'étape a) et au moins une huître fournie à l'étape b) dans une cuve. Par exemple, on peut mettre plusieurs huîtres fournies à l'étape a) avec une huître de l'étape b) dans une cuve et inversement. On peut également mettre plusieurs huîtres fournies à l'étape a) avec plusieurs huîtres fournies à l'étape b) dans une cuve.

De préférence, lesdites huîtres fournies aux étapes a) et b) dans le procédé de l'invention sont isolées deux par deux à l'étape c) du procédé de l'invention, c'est à dire que chaque cuve ne contient que deux huîtres, une correspondant à une huître fournie à l'étape a) et l'autre correspondant à une huître fournie à l'étape b)

La cuve utilisée dans le procédé de l'invention peut être une cuve telle que décrite précédemment.

Les étapes d) à i) peuvent être réalisées avec les moyens de mise en oeuvre décrits précédemment. Ces moyens peuvent être adaptées à chaque étape du procédé de l'invention.

Selon le procédé de l'invention, la maturation effectuée à l'étape d) du procédé de l'invention peut être effectuée par tout moyen connu de l'homme du métier permettant la maturation des huîtres. De préférence la maturation des huîtres est réalisée par augmentation progressive de la photopériode. Cette augmentation peut être réalisée sur une période d'une semaine à 6 mois, de préférence de 1 mois à 3 mois. Le temps d'éclairage minimal est compris entre 1 h à 12 h, le temps d'éclairage maximal peut être compris entre 12 h à 23 h. L'étape de maturation peut être effectuée à toute température permettant la réalisation du procédé de l'invention. De préférence, ladite maturation peut être effectuée avec une augmentation progressive de la température jusqu'à une température de préférence comprise entre 20 et 30°C. De préférence, la maturation est effectuée à une température comprise entre 20 et 30°C. Le choix de la température finale de maturation dépend notamment des souches utilisées lors du procédé de l'hybridation et de leurs réponses à la maturation. La température peut être adaptée en fonction des huîtres à hybrider. Le contrôle de la réponse des huîtres à cette étape peut être réalisé par le contrôle de la maturité des gonades et de la formation des gamètes. Les huîtres présentent différents stades de maturité numérotés de 0, correspondant au repos sexuel, à 4b correspondant à l'émission de gamètes. Le contrôle de la maturité peut être réalisé par des procédés connus de l'homme du métier, par exemple par observation de l'ouverture des valves sous balnéation anesthésique en utilisant du Chlorure de magnésium (MgCl₂). Dans le procédé de l'invention, l'huître est considérée comme mature quand celle-ci a atteint le stade 3 de maturation des gonades.

Pendant cette étape de maturation, l'apport nutritionnel peut être effectué par tout moyen approprié connu de l'homme du métier. Il peut s'agir, par exemple d'un apport nutritionnel tel que défini précédemment. Il peut s'agir par exemple d'algues unicellulaires, de phytoplancton, de bactéries benthiques, de microparticules alimentaires etc.

Dans un mode de réalisation particulier dans lequel la cuve est alimentée par un circuit d'eau, pendant l'étape de maturation, ledit circuit peut être isolé d'une cuve à l'autre. L'isolement du circuit d'eau permet entre autres d'éviter des échanges d'eau de cuve à cuve et donc des échanges de substances, par exemple de protéines et/ou d'agents pathogènes et/ou substances chimiques pouvant être émises par des huîtres matures cultivés dans d'autres cuves. Ces substances peuvent en effet être capables d'orienter le sexe des géniteurs. Les échanges de gamètes émis par des huîtres matures cultivées dans d'autres cuves, indésirables pour la réalisation de l'invention, peuvent provoquer des autofécondations inopportunes entre lignées soeurs.

Selon l'invention, l'induction de l'émission des gamètes telle que définie à l'étape e) du procédé de l'invention est de préférence réalisée sur des huîtres matures par exemple des huîtres au stade 3 de maturation des gonades. Ces huîtres matures peuvent être, par exemple les huîtres obtenues à l'étape d) du procédé de l'invention. L'agent d'induction utilisé à l'étape e) du procédé de l'invention peut être tout moyen d'induction connu de l'homme du métier, par exemple au moins un choc thermique et/ou au moins une protéine par exemple une hormone, une substance chimique ou un neurotransmetteur, par exemple la sérotonine.

De manière préférée, l'agent d'induction peut être un ou plusieurs chocs thermiques. Il correspond à une baisse et augmentation successives de température, la variation de température étant adaptée à la réalisation du procédé de l'invention. Par exemple, les températures maximales peuvent être comprises entre 25°C et 55°C et les températures minimales entre 5°C et 20°C.

L'induction, par exemple le choc thermique permet l'émission des gamètes mâles en premier ce qui stimule la ponte des gamètes femelles. L'induction de l'émission des gamètes femelles est réalisée par voie chimique par les gamètes mâles. Il peut s'agir par exemple d'une hormone ou d'un neurotransmetteur, par exemple la sérotonine.

L'induction de l'émission des gamètes femelles peut être également réalisée par toute substance connue de l'homme du métier induisant l'émission des gamètes femelles. Par exemple, une substance chimique, une hormone ou un neurotransmetteur, par exemple la sérotonine, de préférence de l'eau contenant les gamètes mâles, de l'eau filtrée ayant contenu des gamètes et/ou un milieu de dilution contenant du sperme actif issu de cryoconservation.

Dans un mode de réalisation particulier, l'étape e) d'induction de l'émission des gamètes par les huîtres est de préférence réalisée dans une cuve sans circulation d'eau afin d'éviter la dispersion des gamètes. Dans ce cas, si une circulation d'eau à lieu dans les étapes précédentes, pour cette étape e), on coupe le circuit d'eau.

Dans un autre mode de réalisation particulier de l'étape e), la circulation de l'eau d'une cuve à une autre peut être également isolée afin d'éviter la dispersion de substances telle que des hormones ou des gamètes mâles dans les autres cuves.

Selon le procédé de l'invention, l'étape f) correspond à la fécondation des gamètes femelles par les gamètes mâles, les gamètes mâles étant préalablement filtrées par l'huître femelle. La fécondation a lieu dans la cavité palléale de l'huître. Les moyens de mise en oeuvre de cette étape peuvent être ceux tels que décrit précédemment ou tout moyen connu de l'homme du métier. Ces moyens sont, bien entendu, de préférence, adaptés à la réalisation de la fécondation.

Selon le procédé de l'invention, l'incubation des larves telle que définie à l'étape g) peut être réalisée, de préférence dans la cavité palléale de l'huître. Durant cette étape d'incubation des larves, les moyens de mise en oeuvre de cette étape peuvent être ceux tels que décrits précédemment ou tout moyen connu de l'homme du métier. Ces moyens sont de préférences adaptés à la réalisation de cette incubation. Cette étape d'incubation peut être réalisée pendant 8 à 10 jours. Durant cette étape d'incubation, le nourrissage huîtres et des larves peut être réalisé par exemple par du phytoplancton tel que décrit précédemment ou tout autre nutriment approprié. Après cette période d'incubation, les larves sont émises dans le milieu.

Selon le procédé de l'invention, les étapes e) et g) correspondant à l'induction de la ponte, la fécondation et l'incubation des larves peuvent être réalisées in vitro, hors de la cavité palléale ou in vivo à l'intérieur de la cavité palléale de la femelle. Lorsque l'étape g) est réalisée in vivo, c'est à dire dans la cavité palléale de l'huître femelle, les gamètes sont filtrés, fécondes et incubées dans la cavité palléale de l'huître femelle. L'incubation est réalisée de telle manière qu'elle permet l'expulsion de larves à l'étape g).

In vitro, les gamètes sont obtenus par scarification des gonades (technique du « stripping »), permettant de programmer et de contrôler les stades de développement et la fécondation.

Selon le procédé de l'invention, l'étape h) correspond au nourrissage des larves émises dans le milieu à l'étape g) afin d'obtenir des larves oeillées. Le phytoplancton permettant le nourrissage des larves tel que décrit précédemment peut être tout phytoplancton connu de l'homme du métier permettant la réalisation de cette étape. Les moyens de mise en oeuvre de cette étape peuvent être ceux tels que décrits précédemment ou tout moyen connu de l'homme du métier. Ces moyens sont de préférence adaptés à la réalisation du nourrissage des larves.

Selon le procédé de l'invention l'étape i) correspond à la récolte des larves oeillées obtenues à l'étape h). La récolte des larves oeillées peut être réalisée, par exemple 8 à 10 jours après expulsion des larves. Les moyens de récolte des larves oeillées peuvent être tout moyen connu de l'homme du métier, par exemple des filtres, des tamis présentant des pores avec un diamètre permettant la récolte des larves. Par exemple, les tamis peuvent présenter une taille de pore allant de 10 µm à 180 µm, de préférence de 50 µm à 150 µm. La taille des pores peut être choisie en fonction de la taille des larves émises, celle-ci devant être inférieure à la taille des larves émises.

Selon le procédé de l'invention, l'étape j) correspond à la fixation des larves sur des supports de développement. La fixation peut être réalisée dans des cuves telles que décrites précédemment. Les moyens de mise en oeuvre de cette étape peuvent être ceux tels que décrits précédemment ou tout moyen connu de l'homme du métier. Ces moyens sont de préférence adaptés à la réalisation de la fécondation. Lesdites cuves peuvent comporter au niveau de leur paroi des supports de développement identiques ou différents. Les supports de développement des larves peuvent être tout support permettant la réalisation du procédé de l'invention. Par exemple le support de développement peut être des microbrisures de coquille d'huître, de moules, des microbrisures de verre, et/ou tout matériau, tamisé par exemple entre 250 µm et 500 µm, permettant la fixation des larves obtenues par le procédé de l'invention et l'obtention de naissains libres.

Après l'étape de fixation, les naissains se développent afin d'obtenir au moins une huître hybride. Les moyens de mise en oeuvre de cette étape de développement peuvent être ceux tels que décrits précédemment ou tout moyen connu de l'homme du métier. Ces moyens sont de préférence adaptés au développement des larves.

Selon l'invention, l'hybridation peut être réalisée, par exemple, sur une seule saison de reproduction, par exemple sur une période de six mois durant l'année de première maturité. Les conditions de température et d'alimentation peuvent être choisies afin de permettre à chaque huître d'inverser plusieurs fois son sexe au cours de sa première maturité. Lesdites huîtres étant capables d'émettre de nouveaux gamètes à des intervalles variant de 10 à 70 jours.

Avantageusement, la réalisation du procédé de l'invention la première année de maturité peut permettre de disposer d'une qualité optimale des gamètes et des larves provenant des jeunes huîtres.

Les huîtres hybrides obtenues selon le procédé de l'invention sont de types plein-frères, elles proviennent seulement de parents présentant des polymorphismes génétiques particuliers résultants de leurs identités écologiques et géographiques distincts et complémentaires additionnés par hétérozygotie. Cette hybridation et cette formation d'hétérozygotes permettent d'obtenir des hybrides présentant des caractéristiques de résistance aux agents pathogènes.

L'hybridation selon le procédé de l'invention ne se produit jamais dans la nature, notamment en raison de la biologie de la reproduction du genre *Ostrea* et de l'éloignement géographique des populations sauvages génétiquement distinctes. Le système d'élevage fait appel de préférence aux seuls hybrides F1, les qualités d'hétérosis se perdant très vite aux générations suivantes par la dégénérescence consanguine à laquelle les Ostréidés sont particulièrement vulnérables. Le procédé de l'invention permet donc d'obtenir des huîtres résistantes de première génération.

Les huîtres hybrides résistantes peuvent se développer dans différentes conditions. En fonction de l'hybridation réalisée elles peuvent se développer dans les milieux originels des parents. Par exemple, le croisement d'une huître *Ostrea edulis* résistante de Corse avec une huître non résistante d'Atlantique donne un hybride résistant aux agents pathogènes capable de se développer sur les côtes Atlantiques et également en Méditerrané. Cet aspect de l'invention présente un grand avantage par rapport à l'état de la technique sachant que l'huître résistante de Corse ne peut pas se développer sur les côtes Atlantiques, les huîtres de Corse n'étant pas adaptées par exemple au phénomène de marée et donc au phénomène d'immersion et d'émersion, aux variations du milieu, par exemple la salinité, la température.

Ces huîtres résistantes obtenues par hybridation, grâce à leur résistance et leur capacité d'adaptation au milieu, permettent de réduire les coûts et d'améliorer considérablement la production des huîtres dans les bassins ostréicoles infectés par les agents pathogènes.

Les huîtres résistantes obtenues par le procédé de l'invention présentent en outre des propriétés d'adaptation aux variations climatiques, par exemple le réchauffement de la température du milieu de culture.

De plus les hybrides peuvent présenter une croissance journalière augmentée jusqu'à 30 % par rapport à celle des parents dont elles sont issues. Cet aspect augmente l'intérêt de l'utilisation des hybrides résistants dans les bassins de production ostréicole.

Ces huîtres résistantes obtenues par hybridation peuvent également permettre de repeupler des zones infectées par les agents pathogènes, importés ou non par l'homme par la culture d'huîtres infectées, et de permettre la restauration d'un écosystème marin.

Ces huîtres résistantes obtenues par hybridation peuvent également permettre de régénérer la biodiversité de souches cultivées dans les bassins ostréicoles où la consanguinité devient de plus en plus importante. La consanguinité étant une source de dégénérescence de la souche et une cause de la baisse de la productivité des bassins ostréicoles.

La consanguinité des huîtres peut en effet provoquer une diminution de la résistance des huîtres à des agents pathogènes tel que cela est observé depuis 1920 par la multiplication des phénomènes d'épizooties dans les parcs ostréicoles [16]. Les huîtres hybrides résistantes obtenues selon la présente invention ont des propriétés de résistance accrues aux agents pathogènes viraux, bactériens et/ou parasitaires connus par l'homme du métier. Les propriétés accrues de résistances peuvent être par exemple une amélioration de la réponse immunitaire cellulaire et humorale, en particulier au niveau des défenses hémocytaires . Ces propriétés peuvent être dues, par exemple à la restauration de la diversité génétique résultant de l'hétérozygotie [15].

Les huîtres hybrides résistantes obtenues selon l'invention permettent de résoudre le problème des épizooties récurrentes au sein des parcs ostréicoles, par exemple les épizooties dues à l'augmentation de la température de l'eau ou les épizooties dues au virus de l'herpes 1 des huîtres (« Ostreid Herpesvirus-1 (OsHV-1) »). En effet, la diversité génétique des huîtres obtenues par le procédé de l'invention leur confère une rusticité et une immunité supérieure par rapport au huîtres consanguines permettant ainsi de résister aux infections épisodiques dues aux modifications de l'environnement, et particulièrement aux changements climatiques [10].

D'autres avantages pourront encore apparaître à l'homme du métier à la lecture des exemples ci-dessous donnés à titre illustratif.

### EXEMPLES

### Exemple 1 :Huître résistante Ostrea edulis de l'étang de Diane en Corse

Des Huîtres sauvages ont été collectées dans l'étang de Diane, étang classé depuis les années 1980 comme contaminé par les parasites *Bonamia ostreae* et *Martelias refringens,* et reproduites avec succès en écloserie. Le naissain mis en culture à 0,2 g permet d'obtenir dans l'étang une production expérimentale d'excellente qualité. Ce succès permet de tirer les conclusions suivantes :
- L'absence de pathologie jusqu'à taille de commercialisation en zone contaminée par *Bonamia ostreae* et *Martelia refringens* suggère une résistance aux parasitoses liée à un polymorphisme génétique singulier propre à la souche corse de l'huître plate *Ostrea edulis.*
- L'obtention en tête des lots des calibres n°1 et n°0 à 18 mois à partir de naissains de 0,2 g témoigne du potentiel élevé de croissance de la souche corse. La durée du cycle d'élevage est réduite de plus de 50% par rapport à la durée de l'élevage de la belon en Atlantique.
- L'indice de condition s'élève à 16% (poids de chair comestible par rapport au poids total), niveau supérieur à celui de la belon de Bretagne sud. Les tests gustatifs probants confirment la qualité remarquable de la souche corse au plan commercial.

On a ensuite testé la situation sanitaire et la résistance aux agents pathogènes de la souche. Ces examens sont effectués selon les protocoles prescrits par l'O.I.E (Organisation Mondiale de la Santé Animale pour la bonamiose et la marteiliose) :
- Examen cytologique au microscope de l'empreinte tissulaire du ventricule cardiaque ou des branchies : aucune observation de petits organismes sphériques ou ovoïdes de 2 à 5 µm à l'intérieur des hémocytes après coloration (*Bonamia*).
- Examen histopathologique au microscope de coupes tissulaires de branchies, glandes digestives, gonades fixées et colorées. Pas d'observations de cellules parasitaires de 2 à 5 µm à l'état libre ou à l'intérieur des hémocytes (*Bonamia*). Pas d'anomalie de la glande digestive, ni présence d'organismes sphériques de 20 à 30 µm attestant de la présence de *Martelia.*
- Détection par la technique d'immunofluorescence d'anticorps monoclonaux montrant la résistance de la souche corse face à l'existence des parasites *Bonamia* et *Martelia* dans le milieu naturel.
- Réaction de polymérisation en chaîne (PCR) : aucune détection de *Martelia refringens* avec la sonde ADN spécifique ciblant la région ITS1 du parasite.
- Analyse PCR-RFLP (Restriction fragment length polymorphism) : contrôle négatif de l'ADN extrait de tissus d'huîtres ciblant le fragment SSU rDNA caractéristique du parasite *Bonamia ostreae,* attestant la non contamination des souches corses.
- Technique moléculaire d'hybridation in situ (ISH) : les sondes spécifiques de l'ADN de *Bonamia ostreae,* fragment SSU rDNA, et de *Martelia refringens,* fragment ITS1, sont mises au contact avec des coupes histologiques des tissus de l'huître. Les contrôles négatifs effectués par observation au microscope attestent de l'absence de *B.ostreae* et *M*. *refringens* dans les tissus de la souche corse. Ces examens montrent que les populations sauvages d'huîtres plates de l'étang de Diane sont au contact de *B. ostreae* et *M*. *refringens Bonamia* et ne sont pas infectées par ces parasites dans ce milieu naturel.

On a ensuite effectué des essais d'inoculation artificielle de ces parasites.
- Les huîtres plates de Corse ont été confinées pendant plusieurs mois dans des bacs en cohabitation avec des huîtres fortement contaminées par *Bonamia ostreae.* Les huîtres n'ont pas développé la maladie et ont répondu négativement aux tests de détection décrits ci-dessus.
- Les huîtres de Corse ont reçu une injection dans la cavité palléale de 50 µl d'eau de mer filtrée contenant 5 x 10⁶ parasites viables *Bonamia ostreae* isolés selon la méthode de Mialhe et al. (1988). Auparavant, les huîtres ont été anesthésiées par balnéation avec une solution de 3,5 % MgCl₂-6H₂O permettant l'ouverture des valves.

Après injection de l'inoculum de la dose contaminante dans la cavité palléale, les huîtres sont conservées une heure hors de l'eau avant d'être remises dans les bacs d'élevage en eau de mer aérée par bullage. Au-delà d'un mois d'élevage, aucune mortalité n'est observée et les huîtres répondent négativement aux tests de détection de *Bonamia ostreae* décrits ci-dessus. Cette technique d'infection expérimentale par balnéation ou par injection dans la cavité palléale n'est pas utilisable pour tester la résistance à *Martelia refringens,* en l'absence de transmission horizontale directe de ce parasite.

Ces expérimentations montrent que l'huître plate corso-sarde sauvage, provenant des Etangs de Diane, d'Urbino, golfes de Porto Vecchio et de Santa Manza, lagunes de Sardaigne, est 100% résistante au parasite *Bonamia ostreae,* protozoaire de la famille des Haplosporidies. Ces expérimentations effectuées démentent formellement l'état de la connaissance scientifique, selon lequel la souche autochtone d'*Ostrea edulis* serait décimée par une maladie provoquée par un protozoaire. les mentions erronées figurant dans la littérature (MINICONI R., 2000, Les fruits de mer des côtes de Corse, Editions Alain Piazzola, P. 90 [9]) se référant de fait à des essais d'élevage de naissains importés originaire d'atlantique, non résistants et/ou infectés.

Le recoupement de toutes les observations faites sur l'huître plate de Corse manifeste une résistance de la souche sauvage aux haplosporidies, protozoaires, martelias, parasites, agents pathogènes opportunistes et agents contaminants externes faisant appel aux mécanismes généraux d'immunité cellulaire (hémocytes) et humorale des bivalves, dont le système immunitaire reste non-adaptatif, c'est-à-dire dépourvu de mémoire immunitaire.

D'autres populations sauvages d'huîtres plates de méditerranée ont été testées selon le protocole précédent et ont démontré une résistance totale aux parasites *Bonamia ostreae* et *Martelia refringens.* Il s'agit des populations sauvages de Corse, du Maroc au niveau de la lagune de Nador, de Tunisie au niveau du Golfes de Gabès, de Libye au niveau des lagunes proches de la frontière tunisienne et de Grèce.

### Exemple 2 : Huître résistante Ostrea Edulis de l'étang de Diane en Corse.

Des huîtres sauvages ont été collectées dans l'étang de Diane, zone contaminée par les agents pathogènes *Bonamia ostrae* et *Martelia refringens,* et reproduites en écloserie. Les huîtres sauvages sont prélevées directement sur un site naturel et sont résistantes aux agents pathogènes.

Les huîtres sauvages prélevées sont des huîtres qui présentent un diamètre supérieur à 60mm et qui ont l'age de première reproduction.
Une fois le prélèvement effectué, les huîtres sont conditionnées dans un récipient d'un volume de 20 l, comportant 20 litres d'eau de mer présentant une salinité de 37 ‰, une température de 13°C et un pH de 8,1 et envoyée à l'écloserie. Les huîtres prélevées sont marquées préalablement à leur conditionnement au moyen d'étiquettes collées à la colle époxy.

### Exemple 3 : Huîtres non résistantes

Dans cet exemple, on a choisi une huître de qualité belon (souche « Quiberon », Bretagne Sud) provenant de captage naturel, d'un calibre de 60 mm, n'ayant pas encore maturé. L'huître utilisée pour la réalisation du procédé de l'invention est une huître qui n'exprime pas de pathologies.

### Exemple 4 : Maturation des huîtres pour la fécondation

Après prélèvement des huîtres à hybrider dans l'exemple 2 et 3, celles-ci sont isolées et associées deux par deux, c'est à dire une huître sensible est associée à une huître résistante. On constitue ainsi vingt couples. Les couples ainsi formés sont déposés dans 20 cuves préalablement remplies ayant chacune un volume unitaire de 20 litres. Chaque cuve a été préalablement remplie avec 20 litres d'eau de mer avec une salinité de 37 ‰. Le pH de l'eau est de 8,1. La température initiale de l'eau est de 13°C, et portée en quinze jours à 20°C. L'eau est oxygénée par un système de pompe avec un débit d'air de 20 l/h pour chacune des cuves.

L'alimentation de l'eau pendant la maturation est réalisée par un circuit d'eau fermé et isolé pour chaque cuve. Les caractéristiques physicochimiques de l'eau sont maintenues constantes tout au long de la maturation.

La maturation des huîtres est réalisée sur une période de 15 à 90 jours par augmentation croissante de la photopériode de 9 heures à 15 heures et une augmentation croissante de la température de l'eau atteignant des niveaux de 20 jusqu'à 30°C.

Pendant cette période de maturation, les apports nutritifs sont réalisés par ajout dans le milieu d'algues unicellulaires de la souche *Isochrysis galbana, Skeletonema costatum, Pavlova lutheri, Chaetoceros calcitrans,* provenant de l'écloserie ou de pâtes concentrées réfrigérées de la société SATMAR. L'apport est effectué à volonté, à raison de trois repas par jour, aussi longtemps que l'alimentation des huîtres est observée. L'apport nutritif est complémenté simultanément par des apports de matières organiques, provenant notamment de rejets de bassins d'alevinage de poissons.

La maturation est vérifiée par l'observation des stades de maturité des gonades par les techniques connues de l'homme du métier sous balnéation anesthésique par du chlorure de magnésium (MgCl₂) Gagnaire B., 2005, « Etude des effets de polluants sur les paramètres hémocytaires de l'huître creuse Crassostrea gigas - Interactions entre environnement, mécanismes de défense, et maladies infectieuses », thèse de Doctorat de l'Université de La Rochelle, 412 pp. [10]

Les huîtres sont matures pour l'induction de la ponte des gamètes lorsque la maturation des gonades atteint le stade 3.

### Exemple 5 : Induction de l'émission des gamètes et fécondation des gamètes

L'induction de l'émission des gamètes est réalisée sur des huîtres ayant subies la maturation de l'exemple 4.

L'induction de l'émission des gamètes est réalisée dans les mêmes conditions, en utilisant les mêmes moyens et avec les mêmes couples d'huîtres que dans l'exemple 4.

Préalablement à l'induction, la circulation de l'eau est arrêtée pour éviter la dispersion des gamètes dans le milieu de culture.

L'induction de l'émission des gamètes est obtenue par choc thermique. La température est alternativement baissée et augmentée de 4°C à 7°C à des valeurs minimales comprises entre 23°C et 30°C et des valeurs maximales comprises entre 30°C à 37°C.

Les mâles fraient en premier ce qui induit par voie chimique l'émission des gamètes femelles par les huîtres femelles.

Les spermatozoïdes libérés dans le milieu sont filtrés par la femelle. La fécondation a fieu dans la cavité palléale de celle-ci, qui va incuber les larves pendant huit à dix jours avant de les expulser à l'extérieur à raison d'environ un million par femelle.

Durant l'étape d'incubation des larves dans la cavité palléale, l'eau est remise en circulation. Les larves sont nourries à l'aide de phytoplancton. Le phytoplancton utilisé pour nourrir les larves est composé d'*Isochrysis galbana" Pavlova lutheri, Chaetoceros forma pumilum,* provenant de cultures en masse menées en cuves situées à l'extérieur de l'écloserie, et complété si nécessaire par de la pâte concentrée d'algues provenant de la société SATMAR. La taille du phytoplancton utilisé est de 20 à 40 µm pour les larves aux premiers stades, de 50 à 200 µm à la métamorphose jusqu'au stade adulte. La quantité journalière de phytoplancton ajoutée dans le milieu est ajustée à volonté, en observant la filtration de la nourriture à l'intérieur des cuves, la circulation d'eau étant interrompue au cours des repas.

### Exemple 6 : Récolte et fixation des larves

La récolte de larves émises dans un milieu de l'exemple 5, est réalisée à l'aide d'un tamis. Les conditions de réalisation de la récolte et de la fixation des sont identiques à celle de l'exemple 3. Le tamis utilisé présente des pores d'un diamètre de 120 µm. L'utilisation du tamis permet de récolter les larves présentes dans le milieu de culture. Celles-ci sont regroupées en lot homogène en fonction de leurs parents. Le regroupement est effectué dans des cuves présentant au fond des microbrisures d'huîtres et de moules concassées et calibrées sur tamis entre 250 µm et 500 µm.
Le volume des cuves est de 2 à 5 m³ Les cuves sont remplies avec de l'eau avec une salinité de 37 ‰ un pH de 8,2 et une température de 20 à 25 °C. Les cuves, en circuit fermé, sont oxygénées avec une pompe avec un débit d'air de 2 à 5 m³/h. Après huit à dix jours d'incubation, les larves sont alors fixées sur des supports de développement. La méthode de fixation des larves utilisées est une méthode classique de fixation de larves sur des supports, notamment décrite dans les documentations techniques d'IFREMER, Ifremer, 2007, Fiche aquaculture du 17 mars 2007, « Les écloseries : cas de l'huître creuse » [11] et dans Helm, M.M., Bourne, N., Lovatelli, A., (comp./éd.) Ecloseries de bivalves, Un manuel pratique. FAO document technique sur les pêches N° 471. Rome, FAO. 2006. 184 p. [12]

### Exemple 7 : Huîtres résistantes et non résistantes

Les tableaux ci-dessous représentent d'autres exemples d'huîtres résistantes et non résistantes pour le procédé de l'invention ;
Tableau 1 : *O*. *edulis* résistante de Méditerranée
Tableau 2 : *O*. *edulis* résistante de Méditerranée
Tableau 3 : *O*. *edulis* non résistante de Méditerranée
Tableau 4 : *O*. *edulis* non résistante
Tableau 5 : autres *Ostréidés* non résistantes

**Tableau 1 : Huîtres résistantes Ostrea edulis de Méditerranée**

| ***Espèce*** | **Origine** | **Localisation de la souche** | **Statut** | **Caractères écologiques** | | |
|---|---|---|---|---|---|---|
| | | | | **Etage** | **Température** | **Salinité** |
| ***Ostrea edulis*** | Méditerranée | CORSE (Diane et Urbino) | Sauvage | Infralittoral | Tempérée chaude | Normale |
| ***Ostrea edulis*** | Méditerranée | MAROC (Nador) | Sauvage | Mediolittoral | Subtropicale | Elevée |
| ***Ostrea edulis*** | Méditerranée | LIBYE (lagunes) | Sauvage | Infralittoral | Subtropicale | Elevée |
| ***Ostrea edulis*** | Méditerranée | TUNISIE (Gabès, Kerkennah) | Sauvage | Mediolittoral | Subtropicale | Elevée |
| ***Ostrea edulis*** | Méditerranée | GRECE (Mer Egée) | Sauvage +élevage | Infralittoral | Tempérée chaude | Normale |

**Tableau 2 : huîtres résistantes Ostrea edulis de Méditerranée**

| **Espèce** | **Origine** | **Localisation** | **Statut** | **Caractères écologiques** | | |
|---|---|---|---|---|---|---|
| | | | | **Etage** | **Température** | **Salinité** |
| *Ostrea edulis* | Méditerranée | ESPAGNE, Murcie, mar Menor) | Sauvage | Infralittoral | Tempérée chaude | Normale |
| *Ostrea edulis* | Méditerranée | TURQUIE (Anatolie, Bosphore) | Sauvage | Infralittoral | Tempérée chaude | Normale |
| *Ostrea edulis* | Méditerranée | UKRAINE, Sevastopol, Crimée | Sauvage + élevage | Infralittoral | Tempérée | Normale |
| *Ostrea edulis* | Méditerranée | RUSSIE, GEORGIE, ROUMANIE | Sauvage | Infralittoral | Tempérée | . Normale |
| *Ostrea edulis* | Méditerranée | CROATIE, Est Adriatique | Sauvage | Infralittoral | Tempérée | normale |
| *Ostrea edulis* | Méditerranée | ESPAGNE, Baléares, Minorque | Sauvage | Infralittoral | Tempérée chaude | Normale |
| *Ostrea edulis* | Méditerranée | EGYPTE, Alexandrie | Sauvage | Infralittoral | Subtropicale | Variable |
| *Ostrea edulis* | Méditerranée | France, Camargue, Port St Louis | Sauvage | Infralittoral | Tempérée | Variable |

**Tableau 3 : Huîtres non résistantes Ostrea edulis de MEDITERRANEE**

| ***Espèce*** | **Origine** | **Localisation de la souche** | **statut** | **Caractères écologiques** | | |
|---|---|---|---|---|---|---|
| | | | | ***Etage*** | ***température*** | ***Salinité*** |
| *Ostrea edulis* | Méditerranée | FRANCE, Thau, Languedoc | Sauvage + élevage | Infralittoral | Tempérée chaude | Normale |
| *Ostrea edulis* | Méditerranée | ITALIE, Venise, nord Adriatique | Sauvage + élevage | Infralittoral | Tempérée | Normale |

**Tableau 4 : Huîtres non résistantes Ostrea edulis d'atlantique, du PACIFIQUE et l'océan INDIEN**

| ***Espèce*** | **Origine** | **Localisation de la souche** | **statut** | **Caractères écologiques** | | |
|---|---|---|---|---|---|---|
| | | | | ***Etage*** | ***température*** | ***Salinité*** |
| *Ostrea edulis* | Atlantique | FRANCE, belon, Bretagne sud | Sauvage + élevage | Médiolittoral | Tempérée froide | Variable |
| *Ostrea edulis* | Atlantique | FRANCE, Cancale, Manche | Sauvage + élevage | Médiolittoral | Tempérée froide | Variable |
| *Ostrea edulis* | Atlantique | ESPAGNE, Galice | Sauvage + élevage | Médiolittoral | Tempérée | Normale |
| *Ostrea edulis* | Atlantique | PORTUGAL | Sauvage + élevage | Médiolittoral | Tempérée | Normale |
| *Ostrea edulis* | Atlantique | GRANDE BRETAGNE | Sauvage + élevage | Médiolittoral | Tempérée froide | Variable |
| *Ostrea edulis* | Atlantique | IRLANDE | Sauvage + élevage | Médiolittoral | Tempérée froide | Variable |
| *Ostrea edulis* | Atlantique | PAYS BAS | Sauvage + élevage | Médiolittoral | Tempérée froide | Variable |
| *Ostrea edulis* | Atlantique | DANEMARK | Sauvage + élevage | Médiolittoral | Tempérée froide | Variable |
| *Ostrea edulis* | Atlantique | CANADA et U.S.A. (Est) | Sauvage + élevage | Médiolittoral | Tempérée | Variable |
| *Ostrea edulis* | Pacifique | CANADA, U.S.A. (Ouest) | Sauvage + élevage | Médiolittoral | Tempérée froide | Variable |

**Tableau 5 : Huîtres non résistantes de genre ou d'espèce différents D'Ostrea edulis**

| ***Espèce*** | **Origine** | **Localisation de la souche** | **statut** | **Statut de l'exploitation** |
|---|---|---|---|---|
| *Ostrea denselamellosa* | Pacifique | CHINE, COREE, JAPON | Sauvage + élevage | Espèce en voie d'extinction Exploitation en déclin |
| *Ostrea puelchana* | Atlantique | BRESIL, ARGENTINE | Sauvage + élevage | Exploitation menacée en Argentine |
| *Ostrea folium* | Atlantique O. Indien | Du MAROC au GABON, INDE AUSTRALIE, MALAISIE | Sauvage + élevage | Elevages expérimentaux en Malaisie |
| *Ostrea permollis* | Atlantique | ANTILLES, U.S.A. (Floride, Caroline du nord) | Sauvage + élevage | |
| *Ostrea stentina* | Atlantique O. Indien | CÔTES AFRICAINES + SUD MEDITERRANEE | Sauvage + élevage | Trop petite pour commercialisation |
| *Ostrea angasi* | Pacifique | AUSTRALIE | Sauvage + élevage | Essais d'exploitation en Australie |
| *Ostrea conchaphila* | Pacifique | U.S.A., CANADA (de Californie à l'Alaska) | Sauvage + élevage | Essais d'exploitation |
| *Tiostrea chilensis* | Pacifique | CHILI, NOUVELLE ZELANDE | Sauvage + élevage | Elevage en déclin (parasitoses) |
| *Crassostrea virginica* | Atlantique | CANADA et U.S.A. (Est) | Sauvage + élevage | Parasitoses Elevage et exploitation en déclin |
| *Crassostrea gasar* | Atlantique | AFRIQUE DE L'OUEST | Sauvage + élevage | Essais inaboutis de domestication |
| *Crassostrea rhizophorae* | O. Indien | AFRIQUE DE L'EST | Sauvage + élevage | Essais inaboutis de domestication |

### 8. Autres exemples d'hybridation. :

Les tableaux ci-dessous montrent d'autres exemples dans le cadre de la présente invention concernant les huîtres non résistantes d'Atlantique, du Pacifique et/ou de l'océan Indien.

La réalisation de l'hybridation de l'invention permet l'acquisition de la résistance aux pathologies, dans la perspective de l'exploitation durable d'huîtres domestiques dont les caractères ont préalablement été choisis afin de satisfaire aux contraintes physiques, biologiques et commerciales des milieux d'élevage concernés.

Tableau 6 : exemples de croisements pour l'obtention d'huîtres résistantes

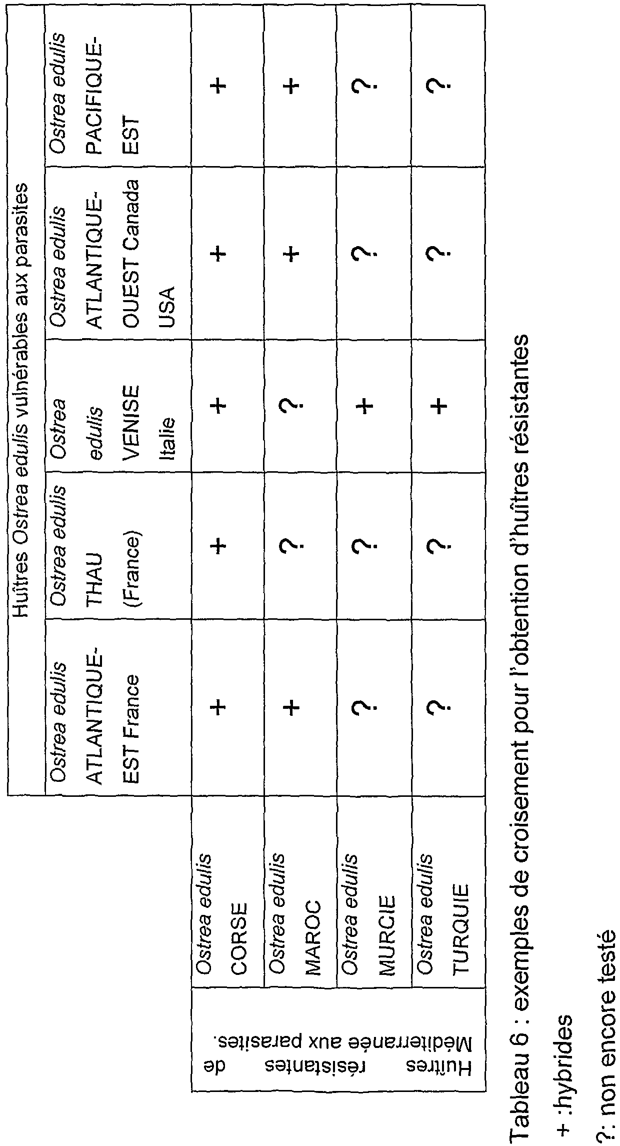

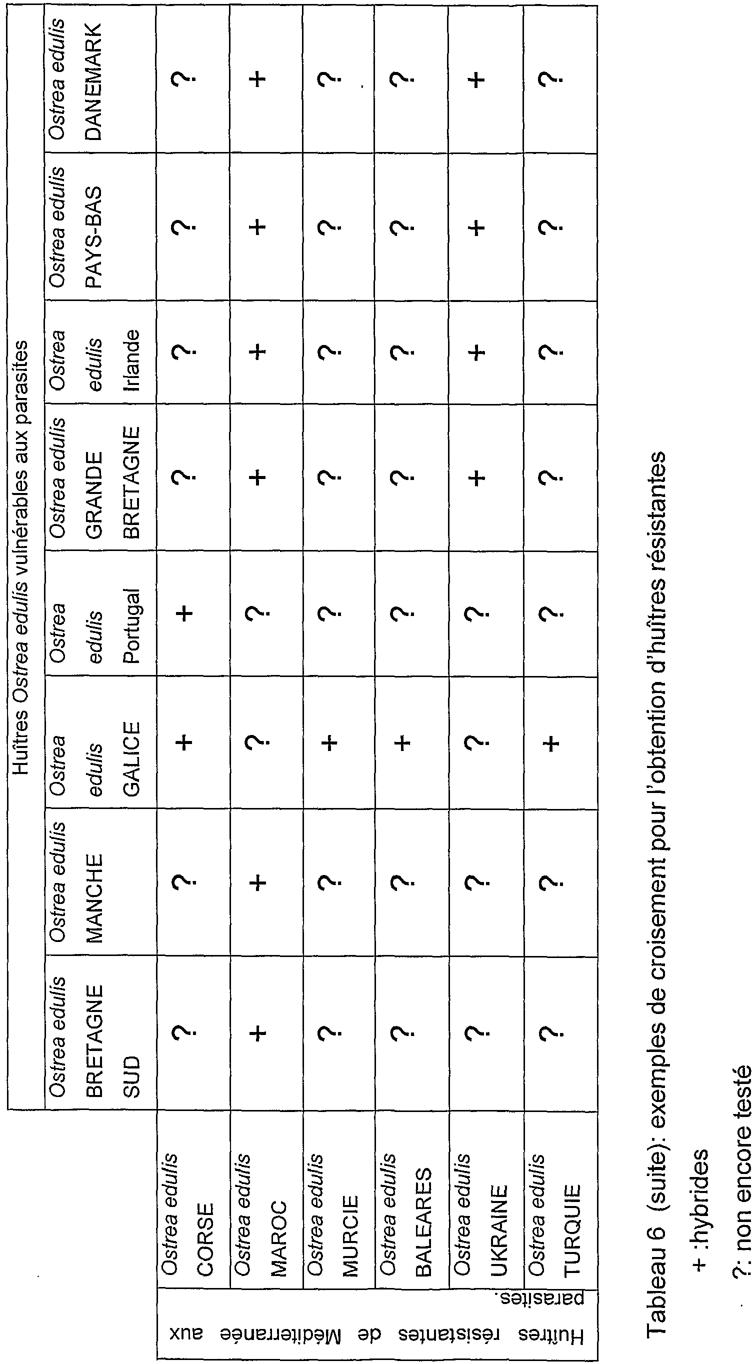

Les hybrides obtenus par le procédé de l'invention tel que présenté précédemment présentent des caractéristiques particulières acquises par cette hybridation qui répondent aux besoins des productions traditionnelles des huîtres plates. Les hybrides sont résistants à la bonamiose et à la marteliose. En outre, ils sont résistants aux agents pathogènes opportunistes. Les hybrides présentent une rusticité et une tolérance à l'amplitude des variations du milieu par exemple l'exondation ou la submersion par les marées, la température, la salinité. Ils ont un fort potentiel de croissance. Ils sont adaptés à la culture en zone intertidale, en eaux riches en nutriments et en matière organique, aux contraintes du réchauffement climatique des eaux littorales. Enfin les hybrides sont tolérants à l'anoxie, à la dessiccation et aux contraintes du transport.

### Exemple 9 : Hybridation entre les huîtres sauvages des étangs de Corse et les huîtres de la lagune de Nador au Maroc.

Les huîtres sauvages des étangs de Corse et des huîtres de la lagune du Nador ont été croisées selon le procédé décrit précédemment, mais hors du cadre de l'invention. Il s'agit dans ce cas d'un croisement entre deux huîtres résistantes. La proximité de Nador par rapport au détroit de Gibraltar est à l'origine de marées pouvant atteindre deux mètres dans la lagune provoque des exondations temporaires des gisements naturels des huîtres marocaines. L'hybridation des huîtres de Diane avec celles de Nador permet de créer des hybrides résistants aux pathologies létales, à forte croissance, adaptés aux contraintes des marées rencontrées en Atlantique, tolérants aux températures croissantes liées au réchauffement climatique, et résistants à la dessiccation lors du transport à sec pour la mise en marché.

La production des naissains hybrides a été comparée à celle de naissains d'huître de Nador produits simultanément en écloserie. L'hybride présente une croissance journalière moyenne supérieure de 30% à celle du lot témoin de naissain marocain, dans le mois qui suit la métamorphose. Par ailleurs, les tailles relatives des naissains hybrides domestiques sont beaucoup plus homogènes que celles du témoin, qui apparaissent très hétérogènes. L'effet d'hétérosis (vigueur hybride) induit par l'hybridation contrôlée se trouve ainsi confirmé dans le cadre de la production d'huîtres plates domestiques à fort potentiel zootechnique.

L'hybride domestique F1 ainsi créé répond donc à la problématique de dégénérescence de l'huître plate « belon » dans les bassins conchylicoles du littoral atlantique.

Les adaptations très larges acquises par cette hybridation selon le procédé de l'invention répondent en conséquence aux besoins des productions traditionnelles des huîtres plates du Portugal, de Galice, de Bretagne, de Normandie, d'Irlande, et des Pays-Bas. Les hybrides sont résistants à la bonamiose et à la marteliose. Ils sont également résistants aux agents pathogènes opportunistes. Les hybrides présentent une rusticité et une tolérance à l'amplitude des marées et des variations du milieu par exemple la température et la salinité. Ils ont un fort potentiel de croissance. Ils sont adaptés à la culture en zone intertidale (élevage sur estran), en eaux riches en nutriments et en matière organique (affinage en abers, affinage en claires), aux contraintes du réchauffement climatique des eaux littorale. Enfin les hybrides sont tolérants à l'anoxie, à la dessiccation et aux contraintes du transport, résistance constatée lors d'envois de colis de Corse vers les marchés bretons.

### Listes des références

[1] Naciri-Graven et al. (1998) « Selecting the flat oyster Ostrea edulis (L.) for survival when infected with the parasite Bonamia ostreae ». J.Exp. Mar Biol. Ecol., 224 :91-107.
[2] N. Taris et al. « Conséquences génétiques de la production de larves d'huîtres en écloserie : étude des processus de dérive et de sélection. » , Les actes du BRG, 6 (2006) 521-541.
[3] Launey S. et al. « Géographie structure in the European flat oyster (Ostrea edulis L.) as revealed by Microsatellite polymorphism ». J Hered. 2002 Sep-Oct;93(5):331-51 (2002).
[4] Diaz-Almela et al. « Reduced female gene flow in the European flat oyster Ostrea edulis ». J Hered. 2004 Nov-Dec;95(6):510-6. (2004).
[5] Lannan J.E., 1971, « Experimental self-fertilization of the Pacific oyster, Crassostrea gigas, utilising cryopreserved sperm », Genetics, 68 : 599-601.
[6] Bougrier et Rabedomanana ,1986. « Cryopreservation of spermatozoa of the Japanese oyster, » Crassostrea gigas. Aquaculture, 58 : 277-280.
[7] Haffray et al. « Domestication et amélioration génétique des cheptels piscicoles dans le cadre du SYSAAF », INRA Prod. Anim., 2004, 17 (3), 243-252.
[8] Gerard A., Naciri-Graven Y., Boudry P., Launey S., Heutebise S., Ledu C., Phelipot P., (1997). Contrôle de la gamétogénèse des huître creuse et plates. In : La reproduction naturelle et contrôlée des Bivalves cultivés en France, Devauchelle N., Barret J., Salaun G., (1997), DRV/RA/RST 97-11, Ifremer Brest, 217 pp.
[9] MINICONI R., (2000) Les fruits de mer des côtes de Corse, Editions Alain Piazzola, p 90.
[10] Gagnaire B., 2005, « Etude des effets de polluants sur les paramètres hémocytaires de l'huître creuse Crassostrea gigas - Interactions entre environnement, mécanismes de défense, et maladies infectieuses », thèse de Doctorat de l'Université de La Rochelle, 412 pp.
[11] Ifremer, 2007, Fiche aquaculture du 17 mars 2007, « Les écloseries : cas de l'huître creuse ».
[12] Helm, M.M., Bourne, N., Lovatelli, A., (comp./éd.) Ecloseries de bivalves, Un manuel pratique. FAO document technique sur les pêches N° 471. Rome, FAO. 2006. 184 p.
[13] Bower, S.M., Mc Giaddery S.E., Price I.M. (1994) Synopsis of infectious diseases and parasites of commercially exploited shellfish. Annual Review of Fish Diseases 4:1-199*.*
[14] Bower, S.M., Mc Gladdery S.E (2003) Synopsis of infectious diseases and parasites of commercially exploited shellfish. URL: http://www-sci.pac.dfo-mpo.gc.ca/sheldis/title_e.htm
[15] Taris N. et al., 2007, Evidence of response to unintentional selection for faster development and inbreeding depression in Crassotrea gigas larvae, Aquaculture, Vol. 272, supplément 1, pages S69-S79, Supplément: Genetics in aquaculture IX.
[16] Anon., 2008, "L'ostréiculture marquée par les épizooties", Le Marin n° 3183, vendredi 11 juillet 2008, 5.

## Revendications

1. Procédé d'obtention d'une huître résistante à des agents pathogènes **caractérisé en ce qu'**il comprend l'hybridation d'une huître *Ostrea edulis* de Méditerranée résistante auxdits agents pathogènes et d'une huître non résistante du sexe opposé d'Atlantique, du Pacifique et/ou de l'océan Indien choisie parmi *Ostrea edulis, Ostrea angasi, Ostrea conchaphila, Ostrea. lurida*, *Ostrea denselamellosa, Ostrea puelchana, Ostrea folium, Ostrea permollis, Ostrea stentina, Tiostrea chilensis, Crassostrea virginica, Crassostrea gasar* et *Crassostrea Rhizophorae,* lesdits agents pathogènes étant responsables d'une maladie choisie dans le groupe comprenant la bonamiose, la marteliose, l'haplosporidose, la microcytose, perkinsose et/ou la irivovirose.

2. Procédé selon la revendication 1, dans lequel l'hybridation comprend les étapes de :
a) fournir une huître *Ostrea edulis* de Méditerranée résistante auxdits agents pathogènes,
b) fournir une huître non résistante auxdits agents pathogènes d'Atlantique, du Pacifique et/ou de l'océan Indien choisie parmi *Ostrea edulis, Ostrea angasi, Ostrea conchaphila, Ostrea. lurida, Ostrea denselamellosa, Ostrea puelchana, Ostrea folium, Ostrea permollis, Ostrea stentina, Tiostrea chilensis, Crassostrea virginica, Crassostrea gasar* et *Crassostrea Rhizophorae* du sexe opposé à ladite huître *Ostrea edulis* résistante,
c) mettre ensemble lesdits huîtres dans une cuve comprenant de l'eau ayant une salinité permettant l'hybridation,
d) maturation desdites huîtres dans ladite cuve, en photopériode croissante avec un apport nutritionnel suffisant pour ladite maturation,
e) induction de l'émission de gamètes mâles au moyen d'un agent d'induction appliqué aux huîtres,
f) fécondation des gamètes femelles par les gamètes mâles émises à l'étape e),
g) incubation des larves pendant 8 à 10 jours,
h) nourrissage des larves issues de l'étape g) par du phytoplancton de façon à obtenir des larves oeillées,
i) récolte des larves oeillées obtenues, et
j) fixation desdites larves sur un support de développement afin d'obtenir au moins une huître résistante auxdits agents pathogènes.

3. Procédé selon la revendication 2, dans lequel les étapes e) à g) sont réalisées in vitro, les gamètes étant obtenues par scarification des gonades.

4. Procédé selon la revendication 2, dans lequel les étapes e) à g) sont réalisées in vivo, l'étape f) de fécondation des gamètes émises à l'étape e) étant réalisée après filtration desdites gamètes dans la cavité palléale de l'huître femelle et l'étape g) d'incubation étant réalisée dans la cavité palléale de l'huître femelle de manière à obtenir une expulsion de larves.

5. Procédé selon l'une quelconque des revendications 2 à 4, dans lequel ladite cuve est alimentée en continu par un circuit de ladite eau et dans lequel pour la mise en oeuvre de l'étape e) ledit circuit d'eau est fermé.

6. Procédé selon la revendication 5, dans lequel ledit circuit d'eau est isolé.

7. Procédé selon l'une quelconque des revendications 2 à 6, dans lequel la maturation à l'étape d) est effectuée à une température de 20 à 30°C.

8. Procédé selon l'une quelconque des revendications 2 à 7, dans lequel ladite cuve est un bac d'éclosion.

9. Procédé selon l'une quelconques des revendications 2 à 8, dans lequel le moyen d'induction tel que défini à l'étape e) est un choc thermique et/ou au moins une protéine.

10. Procédé selon la revendication 9 dans lequel le choc thermique est réalisé par baisse et augmentation successives de température à des valeurs comprises entre 5 et 55°C.

11. Procédé selon l'une quelconque des revendications 2 à 10, dans lequel ledit apport nutritionnel est constitué d'algues unicellulaires *vivantes* cultivées en écloserie.

12. Procédé selon la revendication 11, dans lequel les algues unicellulaires sont choisies dans le groupe comprenant *Isochrysis galbana, Skeletonema costatum, Pavlova lutheri, Chaetoceros calcitrans, Tetraselmis sp.*

13. Procédé selon l'une quelconque des revendications 2 à 12, dans lequel l'étape i) est réalisée 8 à 10 jours après expulsion des larves.

14. Procédé selon l'une quelconque des revendications 2 à 13, dans lequel le support de développement de l'étape i) est choisi dans le groupe comprenant des micro-brisures et/ou un matériau tamisé entre 250 et 500 µm.

15. Procédé selon l'une quelconque des revendications précédentes dans lequel l'huitre *Ostrea edulis* résistante de Méditerranée a l'âge de première reproduction et a un diamètre supérieur à 60mm.

16. Procédé selon l'une quelconque des revendications précédentes dans lequel l'huître *Ostrea edulis* résistante et l'huître non résistante sont choisies à l'issue de la période de repos sexuel.

17. Procédé selon l'une quelconque des revendications précédentes dans lequel l'hybridation s'effectue sur une seule saison de reproduction.

18. Procédé selon l'une quelconque des revendications précédentes dans lequel l'huître non résistante est une souche d'huître provenant de l'atlantique, du pacifique ou de l'océan Indien.

19. Huître obtenue par un procédé selon l'une quelconque des revendications 1 à 18.

## Patentansprüche

1. Verfahren zum Erhalten einer Auster, die gegen pathogene Agenzien resistent ist, **dadurch gekennzeichnet, dass** es die Hybridisierung einer aus dem Mittelmeer stammenden Auster *Ostrea edulis,* die gegen diese pathogenen Agenzien resistent ist, mit einer aus dem Atlantischen Ozean, dem Pazifischen Ozean und/oder dem Indischen Ozean stammenden nichtresistenten Auster des anderen Geschlechts, ausgewählt aus *Ostrea edulis, Ostrea angasi, Ostrea conchaphila, Ostrea. lurida, Ostrea denselamellosa, Ostrea puelchana, Ostrea folium, Ostrea permollis, Ostrea stentina, Tiostrea chilensis, Crassostrea virginica, Crassostrea gasar* und *Crassostrea rhizophorae,* umfasst, wobei die pathogenen Agenzien für eine Krankheit, ausgewählt aus der Gruppe umfassend Bonamiose, Marteliose, Haplosporidiose, Mikrozytose, Perkinsose und/oder Iridovirose, verantwortlich sind.

2. Verfahren nach Anspruch 1, bei dem die Hybridisierung die folgenden Schritte umfasst:
a) Bereitstellen einer aus dem Mittelmeer stammenden Auster *Ostrea edulis,* die gegen die pathogenen Agenzien resistent ist,
b) Bereitstellen einer aus dem Atlantischen Ozean, dem Pazifischen Ozean und/oder dem Indischen Ozean stammenden gegen die pathogenen Agenzien nichtresistenten Auster, ausgewählt aus *Ostrea edulis, Ostrea angasi, Ostrea conchaphila, Ostrea. lurida, Ostrea denselamellosa, Ostrea puelchana, Ostrea folium, Ostrea permollis, Ostrea stentina, Tiostrea chilensis, Crassostrea virginica, Crassostrea gasar* und *Crassostrea rhizophorae,* die das der resistenten Auster *Ostrea edulis* entgegengesetzte Geschlecht aufweist,
c) Zusammengeben der Austern in ein Becken, das Wasser mit einem Salzgehalt, der die Hybridisierung gestattet, umfasst,
d) Heranreifenlassen der Austern in dem Becken unter ansteigender Tageslänge mit einer Nährstoffzufuhr, die für das Heranreifen ausreichend ist,
e) Induzieren der Abgabe von männlichen Gameten mit einem Induktionsmittel, das auf die Austern appliziert wird,
f) Befruchten der weiblichen Gameten durch die in Schritt e) abgegebenen männlichen Gameten,
g) 8- bis 10-tägiges Inkubieren der Larven,
h) Ernähren der in Schritt g) erhaltenen Larven durch Phytoplankton, um zu Larven mit Auge zu gelangen,
i) Ernte der erhaltenen Larven mit Auge, und
j) Sesshaftwerdenlassen der Larven auf einem Entwicklungssubstrat, um zu mindestens einer gegen die pathogenen Agenzien resistenten Auster zu gelangen.

3. Verfahren nach Anspruch 2, wobei die Schritte e) bis g) in vitro durchgeführt werden und die Gameten durch Skarifizieren der Gonaden erhalten werden.

4. Verfahren nach Anspruch 2, wobei die Schritte e) bis g) in vivo durchgeführt werden, Schritt f) der Befruchtung der in Schritt e) abgegebenen Gameten nach Abfiltrieren der Gameten in der Mantelhöhle der weiblichen Austern erfolgt und Schritt g) des Inkubierens in der Mantelhöhle der weiblichen Auster erfolgt, und zwar so, dass Larven ausgestoßen werden.

5. Verfahren nach einem der Ansprüche 2 bis 4, wobei das Becken kontinuierlich durch einen Kreislauf des Wassers ernährt wird, und wobei zur Durchführung von Schritt e) der Wasserkreislauf geschlossen wird.

6. Verfahren nach Anspruch 5, wobei der Wasserkreislauf isoliert wird.

7. Verfahren nach einem der Ansprüche 2 bis 6, wobei das Heranreifen in Schritt d) bei einer Temperatur von 20 bis 30°C erfolgt.

8. Verfahren nach einem der Ansprüche 2 bis 7, wobei es sich bei dem Becken um einen Schlupftank handelt.

9. Verfahren nach einem der Ansprüche 2 bis 8, wobei es sich bei dem wie in Stufe e) definierten Induktionsmittel um einen Temperaturschock und/oder mindestens ein Protein handelt.

10. Verfahren nach Anspruch 9, wobei der Temperaturschock durch Erniedrigen und darauffolgendes Erhöhen der Temperatur auf Werte zwischen 5 und 55°C erfolgt.

11. Verfahren nach einem der Ansprüche 2 bis 10, wobei die Nährstoffzufuhr aus lebenden einzelligen Algen, die in der Schlupfanlage gezüchtet werden, besteht.

12. Verfahren nach Anspruch 11, wobei die einzelligen Algen aus der Gruppe umfassend *Isochrysis galbana, Skeletonema costatum, Pavlova lutheri, Chaetoceros calcitrans, Tetraselmis sp.* ausgewählt sind.

13. Verfahren nach einem der Ansprüche 2 bis 12, wobei Schritt i) 8 bis 10 Tage nach dem Ausstoßen der Larven erfolgt.

14. Verfahren nach einem der Ansprüche 2 bis 13, wobei das Entwicklungssubstrat von Schritt j) aus der Gruppe umfassend Mikrobruchstücke und/oder ein auf zwischen 250 und 500 µm gesiebtes Material ausgewählt ist.

15. Verfahren nach einem der vorhergehenden Ansprüche, wobei die aus dem Mittelmeer stammende resistente Auster *Ostrea edulis* das Erstreproduktionsalter und einen Durchmesser von über 60 mm aufweist.

16. Verfahren nach einem der vorhergehenden Ansprüche, wobei die resistente Auster *Ostrea edulis* und die nichtresistente Auster am Ende der sexuellen Ruheperiode ausgewählt sind.

17. Verfahren nach einem der vorhergehenden Ansprüche, wobei die Hybridisierung in einer einzigen Reproduktionssaison erfolgt.

18. Verfahren nach einem der vorhergehenden Ansprüche, wobei es sich bei der nichtresistenten Auster um einen aus dem Atlantischen Ozean, dem Pazifischen Ozean oder dem Indischen Ozean stammenden Austernstamm handelt.

19. Nach einem Verfahren nach einem der Ansprüche 1 bis 18 erhaltene Auster.

## Claims

1. A method for obtaining oysters resistant to pathogenic agents, **characterized in that** it comprises the hybridization of a Mediterranean oyster *Ostrea edulis* resistant to said pathogenic agents and of a non-resistant oyster of the opposite sex from Atlantic, Pacifica and/or Indian ocean selected among *Ostrea edulis, Ostrea angasi, Ostrea conchaphila, Ostrea luridea, Ostrea denselamellosa, Ostrea puelchana, Ostrea folium, Ostrea stenting Tiostrea chilensis, Crassostrea virginica, Crassostrea gasar* and *Crassostrea Rhizophorae,* said pathogenic agents being responsible for a disease selected among the group comprising bonamiosis, marteliosis, haplosporidosis, microcytosis, perkinsosis and/or irivovirosis.

2. A method according to claim 1, wherein the hybridization includes the following steps:
a) supplying a Mediterranean oyster *Ostrea edulis* resistant to said pathogenic agents,
b) supplying a non-resistant oyster to said pathogenic agents from Atlantic, Pacifica and/or Indian ocean selected among *Ostrea edulis, Ostrea angasi, Ostrea conchaphila, Ostrea luridea, Ostrea denselamellosa, Ostrea puelchana, Ostrea folium, Ostrea stenting Tiostrea chilensis, Crassostrea virginica, Crassostrea gasar* and *Crassostrea Rhizophorae,* the sex of which is opposite to the one of said resistant oyster *Ostrea edulis,*
c) placing together said oysters in a tank containing water having a salinity enabling hybridization,
d) maturation of said oysters in said tank, with an increasing photoperiod with food supply sufficient for said maturation,
e) induction of the emission of male gametes using an induction agent applied to the oysters,
f) fertilization of the female gametes by the male gametes emitted in step e),
g) incubation of larvae for 8 to 10 days,
h) feeding of the larvae obtained in step g) by phytoplankton so as to obtain eyed larvae,
i) collection of the obtained eyed larvae, and
j) fixation of said larvae on a development support in order to obtain at least one oyster resistant to said pathogenic agents.

3. A method according to claim 2, wherein the steps e) to g) are carried out in vitro, the gametes being obtained by scarification of the gonads.

4. A method according to claim 2, wherein the steps e) to g) are carried out in vivo, with the step f) of fertilization of the gametes emitted in step e) being carried out after the filtration of the gametes in the mantle cavity of the female oyster and the step g) of incubation being carried out in the mantle cavity of the female oyster so as to obtain an expulsion of larvae.

5. A method according to any one of claims 2 to 4, wherein said tank is continuously supplied by a circuit of said water and wherein for the implementation of step e) said water circuit is closed.

6. A method according to claim 5, wherein said water circuit is isolated.

7. A method according to any one of claims 2 to 6, wherein the maturation of step d) is carried out at a temperature between 20 and 30°C.

8. A method according to any of claim 2 to 7, wherein said tank is a hatching tank.

9. A method according to any of claim 2 to 8, wherein the induction means such as defined in step e) is a thermal shock and/or at least a protein.

10. A method according to claim 9, wherein the thermal shock is carried out through the successive reduction and increase of the temperature to values between 5 and 55°C.

11. A method according to any of claim 2 to 10, wherein said food supply is composed of living unicellular algae grown in a hatchery.

12. A method according to claim 11, wherein the unicellular algae are selected from the group including *Isochrysis galabana, Skeletonema costatum, Pavlova lutheri, Chaetoceros calcitrans, Tetraselmis sp.*

13. A method according to any of claim 2 to 12, wherein step i) is carried out 8 to 10 days after the expulsion of larvae.

14. A method according to any of claim 2 to 13 wherein the development support of step i) is selected from the group including micro-crushed shells and/or a screened material between 250 and 500 µm.

15. A method according to any of the preceding claim, wherein the resistant oyster *Ostrea edulis* has is of the first farming age and has a diameter above 60 mm.

16. A method according to any of the preceding claim, wherein the resistant oyster *Ostrea edulis* and the non-resistant oyster are selected after the period of sexual latency.

17. A method according to any of the preceding claim, wherein the hybridization is carried out on only one farming season.

18. A method according to any of the preceding claim, wherein the non-resistant oyster is a strain of oyster from the Atlantic, the Pacific or the Indian Ocean.

19. An oyster obtained by a method according to any one of claims 1 to 18.
